# EUROPEAN PATENT APPLICATION

(11) **EP 1 208 857 A1**
(43) Date of publication of application: **29.05.2002**
(21) Application number: 01126476.9
(22) Date of filing: 09.11.2001
(51) Int. Cl.: A61M 5/165

(54) **Filter for infusions**

(30) Priority: 15.11.2000 IT BO000662
(71) Applicant: GVS S.r.l., 40069 Zola Predosa (Bologna) (IT)
(72) Inventor: Scagliarini, Massimo, 40135 Bologna (IT)
(74) Representative: Modiano, Guido, Dr.-Ing.

(57) **Abstract**

A filter for infusions, comprising a flattened box-like body (2) forming a cavity (32) provided with a tubular shank (12) for the inflow of the liquid to be filtered and a tubular shank (15) for the outflow of the filtered liquid, a plate-like element (16) being arranged inside the cavity (32) and having, on its opposite faces, respective pluralities of ridges (19,20) that form channels (23,24), on which two filtration membranes are provided that cover the channels (23,24) and separate them from the cavity (32), the channels (23) being connected to the outflow shank (15) and the cavity (32) being connected to the inflow shank (12).

## Description

The present invention relates to a filter for infusions, particularly for medical infusions such as infusions of lipids, glucosides, et cetera.

Filters for medical infusions of this type are already known. However, they have shortcomings, such as an insufficient filtration surface, constructive complexity and excessive bulk, which make them awkward and troublesome to use.

The aim of the present invention is therefore to provide a filter for infusions in which such constructive and functional drawbacks are substantially reduced.

This aim is achieved with a filter for infusions, characterized in that it comprises a flattened box-like body that forms a cavity provided with a tubular shank for the inflow of the liquid to be filtered and a tubular shank for the outflow of the filtered liquid, a plate-like element being arranged inside said cavity and having, on its opposite faces, respective pluralities of ridges that form channels, on which there are two filtration membranes that cover said channels and separate them from said cavity, said channels being connected to said outflow shank and said cavity being connected to said inflow shank.

Further characteristics and advantages of the present invention will become better apparent from the following description of a preferred but not exclusive embodiment thereof, illustrated only by way of non-limitative example in the accompanying drawing, wherein:
Figure 1 is a front view of the filter;
Figure 2 is a side view of the filter of Figure 1;
Figure 3 is a sectional view, taken along the line III-III of Figure 1;
Figure 4 is a sectional view, taken along the line IV-IV of Figure 1;
Figure 5 is a perspective view of the plate-like element provided inside the box-like body; and
Figure 6 is a perspective view of a closure element of the box-like body.

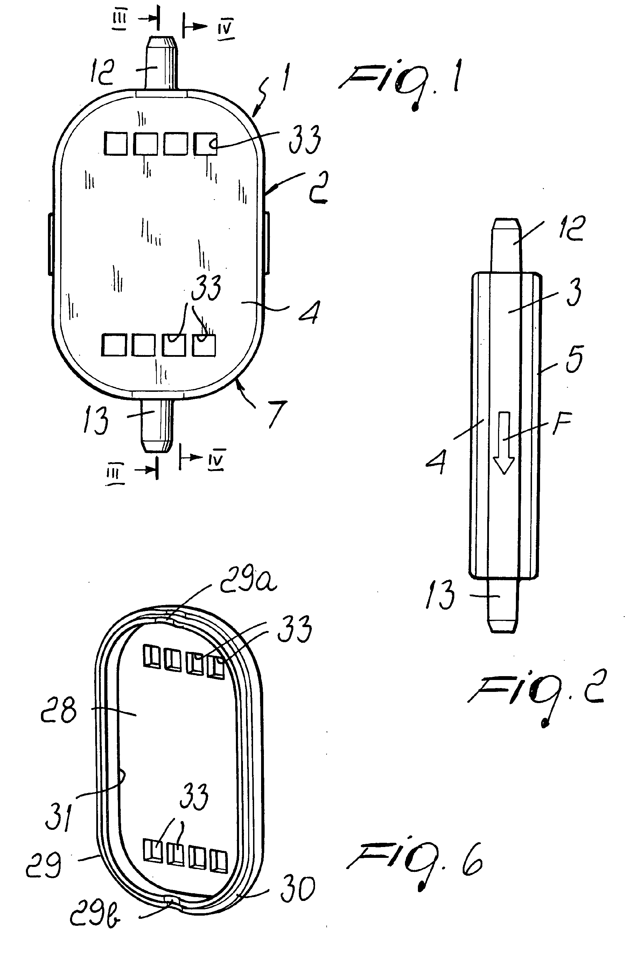

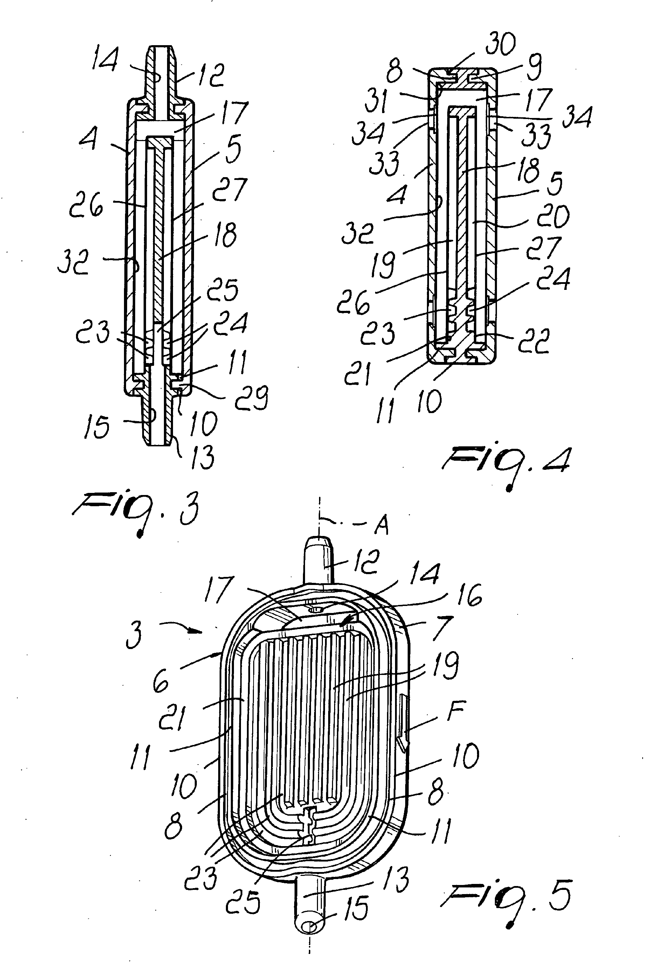

With reference to the figures, the reference numeral 1 generally designates the filter for infusions.

The filter for infusions comprises a flattened box-like body 2, composed of an intermediate part 3 with which two elements 4 and 5 are associated on opposite faces; in relation to their function, which is described hereinafter, they act as a lateral closure of the part 3. The intermediate part 3 and the two elements 4 and 5 are provided by molding plastic material suitable for medical use.

The intermediate part 3 is constituted by a ring-like or annular element 6 that is shaped substantially like a rectangle with rounded corners 7. The annular element 6 has, on its opposite faces, two respective slots 8 and 9 having a rectangular cross-section, each delimited by two rims 10 and 11, the outer rim 10 being lower than the inner rim 11.

Two tubular shanks 12 and 13 are rigidly coupled to the annular element 6, protrude outwardly from midpoints of the shorter sides of the rectangle formed by the annular element 6, and form ducts 14 and 15 that end inside the annular element 6.

The tubular shanks 12 and 13 are coaxial along an axis A that coincides with the intersection line of the two ideal central planes of the box-like body 2.

The annular element 6 surrounds a plate-like element, generally designated by the reference numeral 16, which is perimetrically joined or coupled to the annular element 6 along the two longer sides and the shorter side from which the outlet shank 13 protrudes, and forms or delimits a chamber 17 on the opposite shorter side from which the inflow shank 12 protrudes.

The plate-like element 16 comprises a wall 18, from the opposite faces of which parallel ridges 19 and 20 and perimetric steps 21 and 22, equal in height to the ridges 19 and 20, rise. The ridges 19 and 20 are parallel to the axis A, and the steps 21 and 22 surround the ridges and are adjacent to the internal rim 11.

The parallel ridges 19 and 20 and the longer or longitudinal portions of the steps 21 and 22 form between them channels 23 and 24 which, at the end proximate to the chamber 17, are closed by the shorter or transverse portions of the steps 21 and 22. The channels 23 and 24 merge, at the opposite end, into a passage constituted by a through opening 25 provided in the wall 18 and connected to the duct 15 of the outlet shank 13.

The channels 23 and 24 are closed by respective filtering membranes 26 and 27, which are fixed perimetrically on the steps 21 and 22 and on the crests of the parallel ridges 19 and 20. The filtering membranes 26 and 27 can be fixed in any manner, for example by co-injection during molding of the intermediate part 3 or by ultrasonic welding.

The two closure elements 4 and 5 associated with the intermediate part 3 are mirror-symmetrical and each one comprises a cover 28 whose contour is identical to that of the annular element 6. A lip 29 protrudes from the peripheral region of the cover 28 at right angles thereto; its shape and thickness are complementary to those of the slots 8 and 9 of the annular element 6, so that it can be inserted snugly in said slots. Conveniently, the lip 29 has, at the regions that overlap the shanks 12 and 13, bends 29a and 29b in order to follow the raised bottom of the slots 8 and 9 caused by the need to form the ducts 14 and 15 through the annular element 6.

The lip 29 forms, together with the cover 28, an outer seat 30 and an inner seat 31 which, when the lip 29 is inserted in the respective slot 8, 9, are engaged by the rims 10 and 11.

Once the two closure elements 4 and 5 have been coupled to the annular element 6 and fixed hermetically thereto, for example by ultrasonic welding, a cavity 32 is defined which is connected to the inflow shank 12 through the chamber 17 on one side and is connected to the outlet shank 13 through the filtering membranes 26 and 27, the channels 23 and 24, and the opening 25 on the other side.

The outlet filter is completed by series of openings 33, provided as two series for each cover, at the opposite ends of the covers 28 of the two closure elements 45. The openings 33 are closed by hydrophobic membranes 34 that are applied on the inside; in a known manner, said membranes allow the outflow of any air contained in the infusion liquid but do not allow the outflow of said liquid.

It is evident from the above description that the filter 1, once assembly is complete, assumes a flattened and compact configuration.

During use, the infusion liquid, through the shank 12, enters the chamber 17, from where it expands into the cavity 32, striking and flowing through the filtering membranes 26 and 27 in order to be conveyed by the channels 23 and 24 in the direction of flow F toward the opening 25 and is made to flow out through the outlet shank 13.

The main characteristic of the present invention consists in that the filtering membranes 26 and 27 provide a filtering surface that is comparatively larger than that of conventional filters. Moreover, it should be noted that the ridges 19 and 20 ensure effective support of the membranes without compromising or reducing their filtration capacity. In this regard, the fact is stressed that the plate-like element 16 divides the liquid conveyed by the shank 12 into two streams. In this manner, with respect to conventional filters, the surface of the membranes supported by the ridges and struck by the liquid is greater with respect to the stream that flows through it.

Another advantage is constituted by the surface dimensions of the cavity 32, which ensure a uniform distribution of the filtration pressure over the entire surface of the membranes 26 and 27 in any orientation during use. In this regard, it is noted that the particular arrangement of the membranes inside the box-like body 2 allows to arrange a plurality of series of hydrophobic membranes 34 on both covers, so as to make the outflow of air insensitive to the orientation of the filter. Moreover, the arrangement of the openings 33 on two rows that pass through the covers 4 and 5 allows to collect air also from the arc-like regions of the box-like body.

From a constructive point of view, the flattened shape of the box-like body 2 arid the lack of corners prevent the filter from getting caught during use and handling and prevent it from causing injury to users.

In the practical execution of the invention, all the details may be replaced with other technically equivalent elements. Furthermore, the materials used, as well as the shapes and the dimensions, may be any according to requirements without thereby abandoning the scope of the protection of the appended claims.

The disclosures in Italian Patent Application No. BO2000A000662 from which this application claims priority are incorporated herein by reference.

Where technical features mentioned in any claim are followed by reference signs, those reference signs have been included for the sole purpose of increasing the intelligibility of the claims and accordingly such reference signs do not have any limiting effect on the scope of each element identified by way of example by such reference signs.

## Claims

1. A filter for infusions, **characterized in that** it comprises a flattened box-like body (2) that forms a cavity (32) provided with a tubular shank (12) for the inflow of the liquid to be filtered and a tubular shank (13) for the outflow of the filtered liquid, a plate-like element (16) being arranged inside said cavity (32) and having, on its opposite faces, respective pluralities of ridges (20) that form channels (23, 24), on which there are two filtration membranes (26, 27) that cover said channels and separate them from said cavity, said channels (23, 24) being connected to said outflow shank (13) and said cavity (32) being connected to said inflow shank.

2. The filter according to claim 1, **characterized in that** said box-like body (2) comprises an annular element (6) and two closure covers (4, 5), which are associated on the opposite faces of said annular element (6), so as to form said cavity that accommodates said plate-like element (16).

3. The filter according to claim 2, **characterized in that** said annular element (6) is substantially rectangular, with rounded corners (7), and has on opposite faces respective annular slots (8, 9) that are suitable to receive respective annular lips (29) of said covers (4, 5).

4. The filter according to claim 2 or 3, **characterized in that** said plate-like element (16) is perimetrically coupled to said annular element (6), so as to form, on the side of said inflow shank (12), a chamber (17) that is connected to said shank (12) and to said cavity (32), said plate-like element (16) being provided with a passage (25) that is connected to said outlet shank (13) and is connected to said channels (23, 24).

5. The filter according to claim 4, **characterized in that** said plate-like element (16) has, on opposite sides, steps (21, 22) that lie around said ridges (19, 20) and surround them and on which said membranes (26, 27) are fixed perimetrically.

6. The filter according to claim 5, **characterized in that** said steps (21, 22) are arranged adjacent to said annular element (6).

7. The filter according to one of claims 2 to 6, **characterized in that** said covers (4, 5) have openings (33) closed by hydrophobic membranes (34).

8. The filter according to claim 7, **characterized in that** said openings (33) are arranged along transverse rows and are formed at the opposite ends of said covers (4, 5).
